# EUROPEAN PATENT APPLICATION

(11) **EP 4 166 578 A1**
(43) Date of publication of application: **19.04.2023**
(21) Application number: 21842927.2
(22) Date of filing: 09.07.2021
(51) Int. Cl.: C08F 20/10, C08F 4/00, C08F 2/44, A61K 6/70, A61K 6/79, A61K 6/887

(54) **POLYMERIZATION INITIATOR, KIT FOR PREPARING CURABLE COMPOSITION, CURABLE COMPOSITION, CURED PRODUCT, AND DENTAL MATERIAL**

(30) Priority: 15.07.2020 JP 2020121716
(71) Applicant: Mitsui Chemicals, Inc., Tokyo 105-7122 (JP)
(72) Inventor: TAKAHASHI, Issei, Sodegaura-shi, Chiba 299-0265 (JP)
(74) Representative: Hoffmann Eitle
(86) International application number: PCT/JP2021/025955
(87) International publication number: WO 2022/014492

(57) **Abstract**

A polymerization initiator includes a transition metal compound, a nitrogen-containing compound, and a sulfonyl group-containing compound.

## Description

### TECHNICAL FIELD

The present disclosure relates to a polymerization initiator, a kit for preparing a curable composition, a curable composition, a cured product, and a dental material.

### BACKGROUND ART

In the dental field, a synthetic resin molded product or the like is used to repair a tooth defect. For example, in order to repair a large tooth defect, a curable composition that is referred to as cement is used as a tooth restorative material. In recent years, the range of use of cement has been expanded.

For polymerizing a curable composition for a dental material such as cement, a photopolymerization initiator, a chemical polymerization initiator, or the like can be used.

For example, one of general chemical polymerization initiator systems is a redox polymerization initiator formed of a combination of an oxidizing agent and a reducing agent. Known redox type polymerization initiators include, for example, a polymerization initiator system in which an organic peroxide is used as an oxidizing agent and an aromatic amine compound is used as a reducing agent.

Patent Document 1 describes a paste-state polymerizable composition that is characterized by being formed from a first paste and a second paste, the first paste being composed of: (a) a (meth)acrylate compound in an amount of 15 to 100 parts by weight; (b) a filler in an amount of 0 to 85 parts by weight; (c) one or more selected from a persulfate, a hydroperoxide, or a peroxy ester in an amount of 0.01 to 3 parts by weight with respect to 100 parts by weight in total of the component (a) and the component (b); and (d) benzoyl peroxide in an amount of 0.1 to 3 parts by weight with respect to 100 parts by weight in total of the component (a) and the component (b), and the second paste being composed of: (a') a (meth)acrylate compound in an amount of 15 to 100 parts by weight; (b') a filler in an amount of 0 to 85 parts by weight; and (e) an aromatic tertiary amine in an amount of 0.1 to 5 parts by weight with respect to 100 parts by weight in total of the component (a') and the component (b').

Patent Document 1: Japanese Patent Application Laid-Open (JP-A) No. 2013-209598

### SUMMARY OF INVENTION

### Technical Problem

In the case of polymerizing a monomer by means of using a conventional polymerization initiator, the resulting cured product often becomes to have yellow color. When the cured product is used, for example, as a dental material, there is room for improvement in terms of the color of the cured product.

Patent Document 1 does not make sufficient analysis regarding achievement of both of the polymerizability of the monomer and the color of the resulting cured product.

A problem to be solved by an embodiment of the present disclosure is to provide a polymerization initiator that is capable of favorably improving monomer polymerizability and reducing discoloration of a resultant cured product, a kit for preparing a curable composition that includes the polymerization initiator, a curable composition, a cured product of the curable composition, and a dental material.

### Solution to Problem

Specific means for solving the problem include the following aspects.
<1> A polymerization initiator, including a transition metal compound, a nitrogen-containing compound, and a sulfonyl group-containing compound.
<2> The polymerization initiator according to <1>, in which the sulfonyl group-containing compound contains an aromatic ring.
<3> The polymerization initiator according to <1> or <2>, in which the sulfonyl group-containing compound has a structure represented by the following Formula (1): 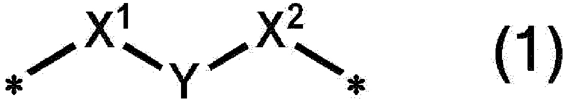
   wherein, in Formula (1, each of X¹ and X² is independently a sulfonyl group or a carbonyl group, and at least one of X¹ or X² is a sulfonyl group, and
   wherein, in Formula (1), Y is an oxygen atom, -NH-, -NNa-, or a sulfur atom, and * represents a bonding position.
<4> The polymerization initiator according to any one of <1> to <3>, in which the transition metal compound includes a copper compound.
<5> The polymerization initiator according to any one of <1> to <4>, in which the nitrogen-containing compound includes an aromatic amine compound.
<6> The polymerization initiator according to <5>, in which the nitrogen-containing compound further includes a heterocyclic amine compound.
<7> The polymerization initiator according to <6>, in which the heterocyclic amine compound is a compound represented by the following Formula (2): 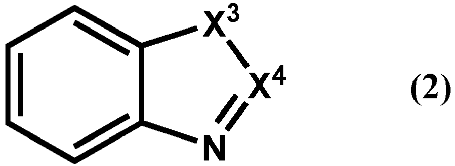 wherein, in Formula (2), X³ is an oxygen atom, a sulfur atom, or a nitrogen atom, and X⁴ is -N= or -CH=.
<8> A kit for preparing a curable composition, including: a first preparation containing a monomer (A); and a second preparation containing a monomer (B), wherein each of the transition metal compound, the nitrogen-containing compound, and the sulfonyl group-containing compound contained in the polymerization initiator according to any one of <1> to <7> is independently contained in at least one of the first preparation or the second preparation.
<9> The kit for preparing a curable composition according to <8>, wherein the first preparation contains the sulfonyl group-containing compound, and the second preparation contains the nitrogen-containing compound.
<10> The kit for preparing a curable composition according to <8> or <9>, wherein the second preparation contains the nitrogen-containing compound and sulfinic acid, and the nitrogen-containing compound includes an aromatic amine compound and a heterocyclic amine compound.
<11> The kit for preparing a curable composition according to any one of <8> to <10>, wherein the total content of organic peroxides contained in the first preparation and the second preparation is 0.10% by mass or less with respect to the total mass of the curable composition to be prepared.
<12> The kit for preparing a curable composition according to any one of <8> to <11>, wherein at least one of the first preparation or the second preparation contains a non-conductive filler.
<13> The kit for preparing a curable composition according to <12>, in which the first preparation contains the non-conductive filler, the content of the non-conductive filler is 10% by mass or more with respect to the total mass of the first preparation, the second preparation contains the non-conductive filler, and the content of the non-conductive filler is 10% by mass or more with respect to the total mass of the second preparation.
<14> The kit for preparing a curable composition according to any one of <8> to <13>, wherein the monomer (A) and the monomer (B) include a (meth)acrylic monomer (C) having a molecular weight of 100 to 2000.
<15> The kit for preparing a curable composition according to <14>, wherein the content of the (meth)acrylic monomer (C) with respect to the total content of the monomer (A) and the monomer (B) is 50% by mass or more.
<16> The kit for preparing a curable composition according to any one of <8> to <15>, which is for a dental material.
<17> A curable composition, including the polymerization initiator according to any one of <1> to <7>, and a monomer.
<18> A cured product of the curable composition according to <17>.
<19> A dental material, including the cured product according to <18>. Advantageous Effects of Invention

According to an embodiment of the present disclosure, it is possible to provide a polymerization initiator that is capable of favorably improving monomer polymerizability and reducing discoloration of a resultant cured product, a kit for preparing a curable composition that includes the polymerization initiator, a curable composition, a cured product of the curable composition, and a dental material.

### DESCRIPTION OF EMBODIMENTS

In the present disclosure, any numerical range indicated using "to" means a range including numerical values described before and after "to" as a minimum value and a maximum value, respectively.

For numerical ranges described in a stepwise manner in the present disclosure, the upper limit value or the lower limit value of one numerical range may be replaced by the upper limit value or the lower limit value of another numerical range in the stepwise description. The upper limit value or the lower limit value of any numerical range described in the present disclosure may also be replaced by a value described in Examples.

In the present disclosure, a combination of two or more preferred aspects is a more preferred aspect.

In a case in which plural substances corresponding to a component of interest are present, the amount of the component described in the present disclosure means the total amount of the plural substances present, unless otherwise specified.

In the present disclosure, "(meth)acryl" encompasses acryl and methacryl, and "(meth)acryloyl" encompasses acryloyl and methacryloyl.

### «Polymerization Initiator»

The polymerization initiator of the present disclosure includes a transition metal compound, a nitrogen-containing compound, and a sulfonyl group-containing compound.

Inclusion of the foregoing compounds in the polymerization initiator of the present disclosure enables the polymerization initiator to favorably improve monomer polymerizability, and to reduce discoloration of a resultant cured product.

### <Nitrogen-containing Compound>

The nitrogen-containing compound is a compound containing nitrogen.

The scope of the nitrogen-containing compound in the present disclosure also includes a salt form.

The nitrogen-containing compound in the present disclosure does not include the sulfonyl group-containing compound described in the present disclosure.

In the present disclosure, for example, a compound containing nitrogen and also containing a sulfonyl group is classified to a sulfonyl group-containing compound.

Examples of the nitrogen-containing compound in the present disclosure include a compound containing an amino group.

Examples of the compound containing an amino group in the present disclosure include an amine compound containing one nitrogen atom, an amine compound containing two nitrogen atoms, and an amine compound containing three or more nitrogen atoms.

Examples of the compound containing an amino group include an aromatic amine compound, an aliphatic amine compound, and a heterocyclic amine compound.

The nitrogen-containing compound in the present disclosure preferably includes an aromatic amine compound, from the viewpoint of improving the polymerizability.

The aromatic amine compound in the present disclosure contains an aromatic hydrocarbon group in the structure thereof. The scope of the aromatic amine compound in the present disclosure does not include a heterocyclic aromatic compound.

The nitrogen-containing compound in the present disclosure preferably further includes an aliphatic amine compound or a heterocyclic amine compound, from the viewpoint of improving storage stability.

An aliphatic amine compound and a heterocyclic amine compound can coordinate with the metal ion of the transition metal compound to trap the metal, and can thereby reduce the reactivity of a composition containing the transition metal compound and the nitrogen-containing compound. As a result, the composition containing the transition metal compound and the nitrogen-containing compound can retain storage stability (that is, it is possible to include the transition metal compound and the nitrogen-containing compound in the same preparation).

When the composition contains a transition metal compound and an aromatic amine, the composition preferably further contain an aliphatic amine compound as a nitrogen-containing compound, from the same viewpoint as that described above.

From the same viewpoint as that described above, the nitrogen-containing compound in the present disclosure preferably further includes a heterocyclic amine compound.

Examples of the aromatic amine compound include:
aromatic primary amine compounds such as aniline and toluidine;
aromatic secondary amine compounds such as aromatic substituted amino acid compounds or salts thereof, typical examples of which include N-methylaniline, N-methyl-p-toluidine, and aromatic substituted glycines such as N-phenylglycine (NPG), N-tolylglycine (NTG), and N,N-(3-methacryloyloxy-2-hydroxypropyl)phenylglycine (NPG-GMA) or alkali metal salts, alkaline earth metal salts, amine salts, and ammonium salts thereof; and
aromatic tertiary amine compounds such as N,N-dimethylaniline (DMA), N,N-dibenzylaniline, N,N-dimethyl-p-toluidine (DMPT), N,N-diethyl-p-toluidine, N,N-di(2-hydroxyethyl)-p-toluidine (DEPT), N,N-dimethyl-p-ethylaniline, N,N-dimethyl-p-isopropylaniline, N,N-dimethyl-p-tert-butylaniline, N,N-dimethylanisidine, N,N-dimethylxylidine, N,N-dimethyl-3,5-di-t-butylaniline, N,N-dimethyl-p-chloroaniline, and N,N-dimethyl-p-fluoroaniline, or salts thereof.

As the aliphatic amine compound, an aliphatic amine compound containing at least one selected from the group consisting of a primary amino group, a secondary amino group, and a tertiary amino group is preferable, and an aliphatic amine compound containing only a tertiary amino group is more preferable.

As the heterocyclic amine compound, a heterocyclic amine compound containing at least one selected from the group consisting of a primary amino group, a secondary amino group, and a tertiary amino group is preferable, and a heterocyclic amine compound containing only a tertiary amino group is more preferable.

Examples of the aliphatic amine compound include EDTA-based (ethylenediaminetetraacetic acid), NTA-based (nitrilotetraacetic acid), DTPA-based (diethylenetriaminepentaacetic acid), HEDTA-based (hydroxyethylethylenediaminetriacetic acid), TTHA-based (triethylenetetraminehexaacetic acid), PDTA-based (1,3-propanediaminetetraacetic acid), DPTA-OH-based (1,3-diamino-2-hydroxypropanetetraacetic acid), HIDA-based (hydroxyethyliminodiacetic acid), DHEG-based (dihydroxyethylglycine), GEDTA-based (glycol ether diaminetraacetic acid), CMGA-based (dialkoxymethylglutamic acid), EDDS-based ((S,S)-ethylenediamine disuccinic acid), and EDTMP-based (ethylenediaminetetra(methylenephosphonic acid), N,N-dimethyl-N',N'-bis(2-dimethylaminoethyl)ethylenediamine (Me6TREN), N,N'-dimethyl-1,2-phenylenediamine, 2-(methylamino)phenol, 3-(methylamino)-2-butanol, N,N'-bis(1,1-dimethylethyl)-1,2-ethanediamine, or N,N,N',N",N"-pentamethyldiethylenetriamine (PMDETA), or salts thereof.

Examples of the heterocyclic amine compound include a heterocyclic amine compound capable of functioning as a monodentate, bidentate, or tridentate heterocyclic electron donor ligand.

Specific examples thereof include heterocyclic amine compounds derived from unsubstituted or substituted heteroarenes such as furan, thiophene, pyrrole, pyridine, bipyridine, picolylimine, γ-pyran, γ-thiopyran, phenanthroline, pyrimidine, bis-pyrimidine, pyrazine, indole, coumarin, thionaphthene, carbazole, dibenzofuran, dibenzothiophene, pyrazole, imidazole, benzimidazole, oxazole, thiazole, bis-thiazole, isoxazole, isothiazole, quinoline, biquinoline, isoquinoline, biisoquinoline, acridine, chroman, phenazine, phenoxazine, phenothiazine, triazine, thianthrene, purine, bisimidazole, and bisoxazoline; or salts thereof.

The aliphatic amine compound or heterocyclic amine compound is preferably N,N-dimethyl-N',N'-bis(2-dimethylaminoethyl)ethylenediamine, 2,2'-bipyridine, N-butyl-2-pyridylmethanimine, 4,4'-di-tert-butyl-2,2'-dipyridine, 4,4'-dimethyl-2,2'-dipyridine, 4,4'-dinonyl-2,2'-dipyridine, N-dodecyl-N-(2-pyridylmethylene)amine, 1,1,4,7,10,10-hexamethyl-triethylenetetramine, N-octadecyl-N-(2-pyridylmethylene)amine, N-octyl-2-pyridylmethanimine, N,N,N',N",N"-pentamethyl-diethylenetriamine, 1,4,8,11-tetracyclotetradecane, N,N,N',N'-tetrakis(2-pyridylmethyl)ethylenediamine, 1,4,8,11-tetramethyl-1,4,8,11-tetraazacyclotetradecane, tris[2-(diethylamino)ethyl]amine or tris(2-methylpyridyl)amine, and more preferably N,N-dimethyl-N',N'-bis(2-dimethylaminoethyl)ethylenediamine (Me6TREN).

The heterocyclic amine compound is preferably a compound represented by the following Formula (2), from the viewpoint of improving the storage stability. 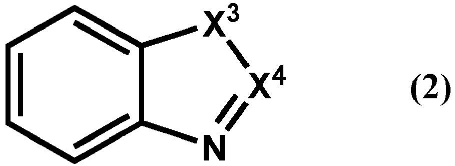 wherein, in Formula (2), X³ is an oxygen atom, a sulfur atom, or a nitrogen atom, and X⁴ is -N= or -CH=.)

### <Sulfonyl group-containing Compound>

The sulfonyl group-containing compound is a compound containing a sulfonyl group.

Inclusion of a sulfonyl group-containing compound in the polymerization initiator of the present disclosure allows the polymerization initiator to improve the polymerizing property.

The sulfonyl group-containing compound preferably satisfies at least one of the following conditions: (i) the sulfonyl group-containing compound includes a carbonyl group; or (ii) the sulfonyl group-containing compound includes two or more sulfonyl groups.

In addition, the sulfonyl group-containing compound preferably satisfies both of the following conditions: (i) the sulfonyl group-containing compound includes a carbonyl group; and (ii) the sulfonyl group-containing compound includes two or more sulfonyl groups.

The polymerization initiator according to any one of claims 1 to 5, wherein the sulfonyl group-containing compound has a structure represented by the following Formula (1). 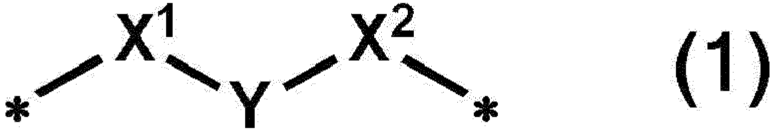
wherein, in Formula (1), each of X¹ and X² is independently a sulfonyl group or a carbonyl group, and at least one of X¹ or X² is a sulfonyl group, and
wherein, in Formula (1), Y represents an oxygen atom, -NH-, -NNa-, or a sulfur atom, and *represents a bonding position.

Preferably, one of X¹ or X² is a sulfonyl group, and the other of X¹ or X² is a carbonyl group.

Y is preferably -NH- or -NNa-, and more preferably -NH-.

In Formula (1), the bonding site represented by * may be directly bonded to an aromatic ring or may be bonded to an aromatic ring via a linking group.

In Formula (1), the two bonding sites represented by * may be bonded to each other via an aromatic ring. For example, in Formula (1), a configuration may be adopted in which the two bonding sites represented by * are bonded to each other via an aromatic ring so that the structure represented by Formula (1) has a fused ring (for example, a 5-membered ring).

The sulfonyl group-containing compound preferably contains an aromatic ring.

The number of aromatic rings contained in the sulfonyl group-containing compound is preferably 1 to 3, more preferably 1 or 2, and still more preferably 1.

The aromatic ring may contain a substituent or may be unsubstituted, and the aromatic ring is preferably unsubstituted.

When the aromatic ring contains a substituent, examples of the substituent include an alkyl group and a carboxy group. Among the above, the substituent is preferably an alkyl group, and more preferably a methyl group.

Examples of the aromatic ring include a benzene ring.

Here, a group containing a sulfonyl group (for example, the group represented by Formula (1)) is excluded from the scope of the above substituent.

Examples of the sulfonyl group-containing compound include the following compounds, but the scope of the sulfonyl group-containing compound in the present disclosure is not limited thereto.

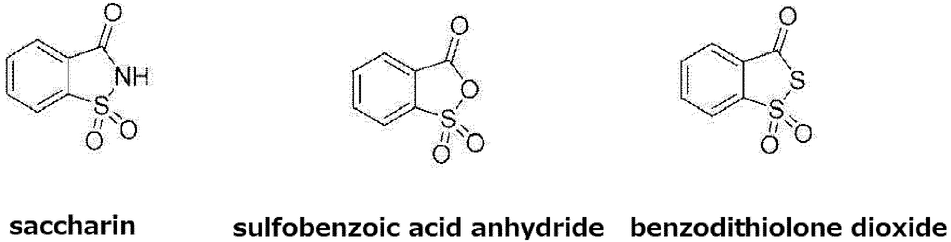

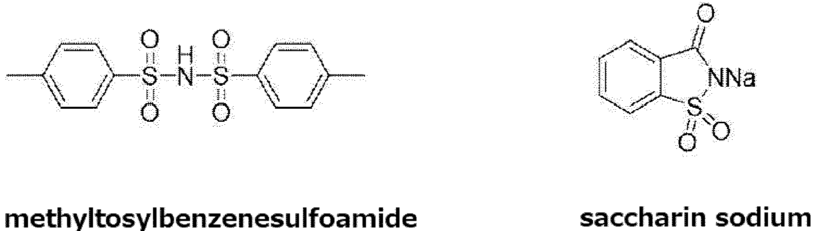

Among the above, saccharin and saccharin sodium are preferable, and saccharin is more preferable, as the sulfonyl group-containing compound.

### <Transition Metal Compound>

The polymerization initiator of the present disclosure includes a transition metal compound.

The transition metal compound is preferably soluble in the monomer component contained in the kit for preparing a curable composition described below.

Examples of the transition metal compound include a copper compound, a vanadium compound, a molybdenum compound, a scandium compound, a titanium compound, a chromium compound, a manganese compound, an iron compound, a cobalt compound, and a nickel compound.

Among the above, the transition metal compound preferably includes at least one of a copper compound or a vanadium compound, and more preferably includes a copper compound.

Examples of the copper compound include: copper carboxylates such as copper acetate, copper isobutyrate, copper gluconate, copper citrate, copper phthalate, copper tartrate, copper oleate, copper octylate, copper octenoate, copper naphthenate, copper methacrylate, and copper 4-cyclohexylbutyrate; β-diketone coppers such as acetylacetone copper, trifluoroacetyl acetone copper, hexafluoroacetylacetone copper, 2,2,6,6-tetramethyl-3,5-heptanedionato copper, benzoylacetone copper; β-ketoester coppers such as copper ethylacetoacetate; copper alkoxides such as copper methoxide, copper ethoxide, copper isopropoxide, copper 2-(2-butoxyethoxy) ethoxide, copper 2-(2-methoxyethoxy) ethoxide; copper dithiocarbamates such as copper dimethyldithiocarbamate; salts of copper and an inorganic acid such as copper nitrate; and copper chloride.

These can be used singly, or in combination of two or more thereof, as appropriate.

Among them, copper carboxylate, β-diketone copper, and β-ketoester copper are preferable, and copper acetate and acetylacetone copper are more preferable, from the viewpoint of solubility in monomers and reactivity.

Examples of the vanadium compound include vanadyl acetylacetonate, vanadium (III) naphthenate, vanadyl stearate, vanadium benzoyl acetonate, bis(maltolato) oxovanadium (IV), and oxobis(1-phenyl-1,3-butanedionato) vanadium (IV).

From the viewpoint of curing property, the blending amount of the transition metal compound is preferably 0.00005 parts by mass to 0.1 parts by mass, more preferably 0.0001 parts by mass to 0.05 parts by mass, and still more preferably 0.001 parts by mass to 0.03 parts by mass, with respect to 100 parts by mass of the total amount of monomer components contained in the kit of the present disclosure for preparing a curable composition.

### <<Kit for Preparing Curable Composition>>

The kit of the present disclosure for preparing a curable composition includes a first preparation containing a monomer (A) and a second preparation containing a monomer (B), and each of the transition metal compound, the nitrogen-containing compound, and the sulfonyl group-containing compound contained in the polymerization initiator of the present disclosure is independently contained in at least one of the first preparation or the second preparation.

### <Monomer>

In the kit of the present disclosure for preparing a curable composition, the first preparation contains a monomer (A), and the second preparation contains a monomer (B).

The monomer (A) and the monomer (B) may be the same monomer or different monomers.

As the monomer (A) and the monomer (B), known monomers can be used. Each of the monomer (A) and the monomer (B) may be a monomer that does not contain an acidic group or may be a monomer that contains an acidic group.

The monomer (A) and the monomer (B) preferably include a monomer that does not contain an acidic group.

The monomer is a monomer that forms a high molecular compound by undergoing a radical polymerization reaction advanced by the polymerization initiator of the present disclosure.

The number of monomers configuring the monomer as used in the present disclosure is not limited to one, and may be two or more. Examples of the monomer that does not contain an acidic group include a (meth)acrylate monomer that does not contain an acidic group. Examples of the (meth)acrylate monomer that does not contain an acidic group include a monofunctional monomer, a bifunctional monomer, and a tri- or higher functional monomer.

In the present disclosure, the monomer content (that is, the total amount of the monomer (A) and the monomer (B) in the curable composition to be prepared) is preferably from 10% by mass to 90% by mass, more preferably from 20% by mass to 75% by mass, and still more preferably from 30% by mass to 60% by mass, with respect to the total mass of the curable composition to be prepared.

Examples of the monofunctional monomer include 2-hydroxyethyl (meth)acrylate, 3-hydroxypropyl (meth)acrylate, 2-hydroxypropyl (meth)acrylate, 1,3-dihydroxypropyl (meth)acrylate, and 2,3-dihydroxypropyl (meth)acrylate. Among them, 2-hydroxyethyl methacrylate (HEMA) is preferable.

Examples of an aromatic compound-based bifunctional monomer include 2,2-bis((meth)acryloyloxyphenyl)propane, 2,2-bis[4-(3-(meth)acryloyloxy)-2-hydroxypropoxyphenyl]propane, 2,2-bis(4-(meth)acryloyloxyethoxyphenyl)propane, 2,2-bis(4-(meth)acryloyloxypolyethoxyphenyl)propane, and 2,2-bis(4-(meth)acryloyloxypolypropoxyphenyl)propane.

Among them, 2,2-bis[4-(3-(methacryloyloxy)-2-hydroxypropoxyphenyl)propane (commonly called "Bis-GMA") and 2,2-bis(4-methacryloyloxypolyethoxyphenyl)propane are preferable.

Examples of an aliphatic compound-based bifunctional monomer include erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl) dimethacrylate (UDMA), and 1,2-bis(3-methacryloyloxy-2-hydroxypropyloxy)ethane.

Among them, glycerol dimethacrylate, triethylene glycol dimethacrylate (TEGDMA), 1,6-hexanediol dimethacrylate (HexDMA), neopentyl glycol dimethacrylate (NPG), 2,2,4-trimethylhexamethylene bis(2-carbamoyloxyethyl) dimethacrylate (UDMA), and 1,2-bis(3-methacryloyloxy-2-hydroxypropyloxy)ethane are preferable.

Examples of the tri- or higher functional monomer include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol] tetramethacrylate, and 1,7-diacryloyloxy-2,2,6,6-tetraacryloyloxymethyl-4-oxyheptane.

Any one of the above-listed monomers may be added singly, or a combination of two or more of the above-listed monomers may be added.

The blending amount of the monomer that does not contain an acidic group is preferably in a range of from 10 parts by mass to 100 parts by mass, more preferably in a range of 20 parts by mass to 100 parts by mass, and still more preferably in a range of 50 parts by mass to 100 parts by mass, with respect to 100 parts by mass of the total amount of monomer components contained in the kit of the present disclosure for preparing a curable composition.

When the monomer component in the kit of the present disclosure for preparing a curable composition contains the below-described acidic group-containing monomer, the blending amount of the monomer that does not contain an acidic group is preferably from 10 parts by mass to 99 parts by mass, more preferably from 30 parts by mass to 97 parts by mass, and still more preferably from 50 parts by mass to 95 parts by mass, with respect to 100 parts by mass of the total amount of the monomer components contained in the kit of the present disclosure for preparing a curable composition.

The monomer (A) and the monomer (B) preferably include a (meth)acrylic monomer (C) having a molecular weight of 100 to 5000.

The molecular weight of the (meth)acrylic monomer (C) is more preferably 120 to 3000, still more preferably 150 to 2000, and particularly preferably 200 to 1000.

The content of the (meth)acrylic monomer (C) with respect to the total content of the monomer (A) and the monomer (B) is preferably 50% by mass or more, more preferably 70% by mass or more, and still more preferably 90% by mass or more.

The content of the (meth)acrylic monomer (C) with respect to the total content of the monomer (A) and the monomer (B) may be 98% by mass or less, or may be 95% by mass or less.

### <Acidic group-containing Monomer>

In the kit of the present disclosure for preparing a curable composition, at least one of the first preparation or the second preparation may contain an acidic group-containing monomer.

When the kit of the present disclosure for preparing a curable composition is used for dental applications, for example, the configuration in which at least one of the first preparation or the second preparation contain an acidic group-containing monomer can impart excellent tooth quality and high adhesiveness to dental prosthetic materials.

Examples of the acidic group-containing monomer include monomers having at least one acidic group and at least one polymerizable group, wherein examples of the acidic group include a phosphoric acid group, a pyrophosphoric acid group, a thiophosphoric acid group, a phosphonic acid group, a sulfonic acid group, and a carboxylic acid group, and examples of the polymerizable group include an acryloyl group, a methacryloyl group, a vinyl group, and a styrene group.

The acidic group-containing monomer has affinity with an adherend and has a tooth decalcification effect.

Examples of a phosphoric acid group-containing monomer include (meth)acryloyloxyalkyl dihydrogen phosphates such as 10-(meth)acryloyloxydecyl dihydrogen phosphate (MDP), bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethyl phenyl hydrogen phosphate, 2-(meth)acryloyloxyethyl-2-bromoethyl hydrogen phosphate, bis[2-(meth)acryloyloxy-(1-hydroxymethyl)ethyl] hydrogen phosphate, and acid chlorides, alkali metal salts, and ammonium salts thereof.

Examples of a pyrophosphoric acid group-containing monomer include bis[2-(meth)acryloyloxyethyl] pyrophosphate, bis[4-(meth)acryloyloxybutyl] pyrophosphate, and acid chlorides, alkali metal salts, and ammonium salts thereof.

Examples of a thiophosphoric acid group-containing monomer include 2-(meth)acryloyloxyethyl dihydrogen thiophosphate, 3-(meth)acryloyloxypropyl dihydrogen thiophosphate, and acid chlorides, alkali metal salts, and ammonium salts thereof.

Examples of a phosphonic acid group-containing monomer include 2-(meth)acryloyloxyethylphenylphosphonate, 5-(meth)acryloyloxypentyl-3-phosphonopropionate, and acid chlorides, alkali metal salts, and ammonium salts thereof.

Examples of a sulfonic acid group-containing monomer include 2-(meth)acrylamide-2-methylpropane sulfonic acid, styrene sulfonic acid, and 2-sulfoethyl (meth)acrylate.

Examples of a carboxylic acid group-containing monomer include a monomer that contains one carboxy group in a molecule thereof, and a monomer that contains plural carboxy groups in a molecule thereof.

Examples of the monomer that contains one carboxy group in a molecule thereof include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides thereof.

Examples of the monomer that contains plural carboxy groups in a molecule thereof include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy) propyl succinate, dihydroxyethyl methacrylate trimethylhexyl dicarbamate, and acid anhydrides or acid halides thereof.

Among the acidic group-containing monomers described above, 10-(meth)acryloyloxydecyl dihydrogen phosphate (MDP), 1,3-di(meth)acryloyloxypropyl dihydrogen phosphate, 2-(meth)acryloyloxyethyl dihydrogen phosphate, 4-(meth)acryloyloxyethyl trimellitate anhydride, 4-(meth)acryloyloxyethyl trimellitate, 2-(meth)acrylamide-2-methylpropane sulfonic acid, 11-(meth)acryloyloxyundecane-1,1-dicarboxylic acid, and dihydroxyethyl methacrylate trimethylhexyl dicarbamate are preferable from the viewpoint that they have a high adhesive power to an adherend.

The acidic group-containing monomer described above may be used singly or in combination of two or more thereof.

The blending amount of the acidic group-containing monomer is preferably from 1 part by mass to 50 parts by mass, more preferably from 3 parts by mass to 40 parts by mass, and still more preferably from 5 parts by mass to 30 parts by mass, with respect to 100 parts by mass of the total amount of monomer components contained in the kit of the present disclosure for preparing a curable composition.

When the blending amount of the acidic group-containing monomer is 1 part by mass or more, high adhesiveness to various adherends can easily be obtained.

When the blending amount of the acidic group-containing monomer is 50 parts by mass or less, the balance between polymerizability and adhesiveness is easy to maintain. The total amount of monomer components means the total amount of the acidic group-containing monomer and the above-described monomer that does not contain an acidic group.

As the monomers in the present disclosure, monomers described in known documents such as WO 2012/157566, WO 2015/015220, WO 2015/015221, and JP-A No. 2016-094482, for example, can be used.

### <Peroxide>

In the kit of the present disclosure for preparing a curable composition, at least one of the first preparation or the second preparation may contain a peroxide.

Examples of the peroxide include organic peroxides and inorganic peroxides, and the peroxide in the present disclosure preferably includes an organic peroxide.

The organic peroxide is not particularly limited, and a known organic peroxide can be used. Typical examples of the organic peroxide include hydroperoxides, peroxyesters, ketone peroxides, peroxyketals, dialkyl peroxides, diacyl peroxides, and peroxydicarbonates. Among them, hydroperoxides are preferable from the viewpoint that use of hydroperoxides provides a small fluctuation in the length of time during which processing is possible even after the curable composition is provided in the dispensed form and stored for a long period of time. The organic peroxide may be used singly or in combination of two or more thereof.

More specific examples of the hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide.

As a peroxyester, a known peroxyester that contains an acyl group on one side of a peroxy group (-OO- group) and a hydrocarbon group (or a group similar thereto) on the other side of the peroxy group can be used without any limitation. Specific examples thereof include α,α-bis(neodecanoylperoxy) diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethyl peroxy-2-ethylhexanoate, t-hexyl peroxy-2-ethylhexanoate, t-butyl peroxy-2-ethylhexanoate, t-butyl peroxyisobutyrate, t-hexylperoxy isopropyl monocarbonate, t-butyl peroxymaleate, t-butyl peroxy-3,5,5-trimethylhexanoate, t-butyl peroxylaurate, 2,5-dimethyl-2,5-bis(m-toluoylperoxy)hexane, t-butylperoxy isopropyl monocarbonate, t-butylperoxy 2-ethylhexyl monocarbonate, t-hexyl peroxybenzoate, 2,5-dimethyl-2,5-bis(benzoylperoxy)hexane, t-butyl peroxyacetate, t-butylperoxy m-toluoylbenzoate, t-butyl peroxybenzoate, and bis(t-butylperoxy) isophthalate. These can be used singly, or in combination of two or more thereof, as appropriate.

Examples of the ketone peroxides include methyl ethyl ketone peroxide, cyclohexanone peroxide, methyl cyclohexanone peroxide, methyl acetoacetate peroxide, and acetylacetone peroxide.

Examples of the peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl4,4-bis(t-butylperoxy) valerate, and 2,2-bis(4,4-di-t-butylperoxycyclohexyl)propane.

Examples of the dialkyl peroxide include α,α-bis(t-butylperoxy)diisopropylbenzene, dicumyl peroxide, 2,5-dimethyl-2,5-bis(t-butylperoxy)hexane, t-butylcumyl peroxide, di-t-butyl peroxide, and 2,5-dimethyl-2,5-bis(t-butylperoxy)hexane-3.

Examples of the diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxides.

Examples of the peroxydicarbonate include di-n-propyl peroxydicarbonate, diisopropyl peroxydicarbonate, bis(4-t-butylcyclohexyl) peroxydicarbonate, di-2-ethoxyethyl peroxydicarbonate, di-2-ethylhexyl peroxydicarbonate, di-2-methoxybutyl peroxydicarbonate, and di(3-methyl-3-methoxybutyl) peroxydicarbonate.

From the viewpoint of curability, the blending amount of the organic peroxide is preferably from 0.01 parts by mass to 1 part by mass, and more preferably from 0.05 parts by mass to 0.5 parts by mass, with respect to 100 parts by mass of the total amount of monomer components contained in the curable composition of the present disclosure.

Examples of the inorganic peroxides include peroxodisulfates and peroxodiphosphates, and, among these, peroxodisulfates are preferable from the viewpoint of curability. Specific examples of the peroxodisulfate include sodium peroxodisulfate, potassium peroxodisulfate, aluminum peroxodisulfate, and ammonium peroxodisulfate.

The peroxodisulfate may be used singly or in combination of two or more thereof. Among the peroxodisulfates described above, sodium peroxodisulfate, potassium peroxodisulfate, and ammonium peroxodisulfate are preferable.

### <Filler>

In the kit of the present disclosure for preparing a curable composition, at least one of the first preparation or the second preparation may contain a filler.

The filler may be added singly, or a combination of two or more fillers may be added. Examples of the filler include an inorganic filler, an organic filler, and a composite filler composed of an inorganic filler and an organic filler.

Examples of the inorganic filler include silica; silica-based minerals such as kaolin, clay, isinglass, and mica; and ceramics and glasses that contain silica as a base material and further contain, for example, Al₂O₃, B₂O₃, TiO₂, ZrO₂, BaO, La₂O₃, SrO, ZnO, CaO, P₂O₅, Li₂O, and/or Na₂O.

Preferable glasses for use include lanthanum glass, barium glass, strontium glass, soda glass, lithium borosilicate glass, zinc glass, fluoroaluminosilicate glass, borosilicate glass, and bioglass.

Crystal quartz, hydroxyapatite, alumina, titanium oxide, yttrium oxide, zirconia, calcium phosphate, barium sulfate, aluminum hydroxide, sodium fluoride, potassium fluoride, sodium monofluorophosphate, lithium fluoride, and ytterbium fluoride are also suitable for use.

More specifically, fine particle silica having a primary particle size of 0.001 µm to 0.1 µm is preferable for use, in terms of adhesive power and handleability. Examples of commercially available products include AEROSII, OX 50, AEROSII, 50, AEROSII, 200, AEROSIL 380, AEROSIL R 972, and AEROSIL 130 (all of which are manufactured by Nippon Aerosil Co., Ltd.).

Examples of the organic filler include polymethyl methacrylate, polyethyl methacrylate, a polymer of polyfunctional methacrylate, polyamide, polystyrene, polyvinyl chloride, chloroprene rubber, nitrile rubber, and styrene-butadiene rubber.

Examples of the composite filler composed of an inorganic filler and an organic filler include a composite filler that includes an inorganic filler dispersed in an organic filler, and an inorganic/organic composite filler that includes an inorganic filler coated with any of various polymers.

In order to improve curability, mechanical strength, and handleability, the filler may be surface-treated with a known surface treatment agent, such as a silane coupling agent, in advance, before use. Examples of the surface treatment agent include vinyltrimethoxysilane, vinyltriethoxysilane, vinyltrichlorosilane, vinyltri(β-methoxyethoxy) silane, γ-methacryloyloxypropyltrimethoxysilane, γ-glycidoxypropyltrimethoxysilane, γ-mercaptopropyltrimethoxysilane, and γ-aminopropyltriethoxysilane.

The blending amount of the filler is preferably in a range of from 10% by mass to 80% by mass, more preferably in a range of from 30% by mass to 80% by mass, and still more preferably in a range of from 50% by mass to 75% by mass, with respect to the total mass of the curable composition of the present disclosure.

### (Non-conductive Filler)

In the kit of the present disclosure for preparing a curable composition, at least one of the first preparation or the second preparation preferably contains a non-conductive filler.

The non-conductive filler means a filler having a resistance value of 1.00 × 10⁻⁴ Ωm or more.

The upper limit of the resistance value of the non-conductive filler is not particularly limited, and may be, for example, 1.00 × 10²⁰ Qm.

Examples of the material of the non-conductive filler include organic substances such as polyethylene, polystyrene, a phenol resin, an epoxy resin, an acrylic resin, and a benzoguanamine resin; inorganic substances such as silicas (such as silica dimethyl silylate), silicates (borosilicate glasses (such as barium borosilicate glass), aluminosilicate glasses (such as boroaluminosilicate glass, strontium boroaluminosilicate glass, fluoroaluminosilicate glass, and barium aluminosilicate glass)), ceramic, boron nitride, and barium nitride.

Among the above materials, silicas and silicates are preferable as the material of the non-conductive filler, and silica dimethyl silylate and barium aluminosilicate are more preferable.

Preferably, the first preparation contains a non-conductive filler, and the content of the non-conductive filler in the first preparation is 10% by mass or more with respect to the total mass of the first preparation; the content of the non-conductive filler is more preferably 20% by mass or more, and still more preferably 30% by mass or more, with respect to the total mass of the first preparation.

The content of the non-conductive filler may be 99% by mass or less, or 95% by mass or less, with respect to the total mass of the first preparation.

When a non-conductive filler is contained in the second preparation, it is preferable that the second preparation contains the non-conductive filler at content of 10% by mass or more with respect to the total mass of the second preparation; the content of the non-conductive filler in the second preparation is more preferably 20% by mass or more, and still more preferably 30% by mass or more, with respect to the total mass of the second preparation.

The content of the non-conductive filler may be 99% by mass or less, or 95% by mass or less, based on the total mass of the second preparation.

In the kit of the present disclosure for preparing a curable composition, the content of the non-conductive filler in the first preparation is, if contained in the first preparation, preferably 10% by mass or more with respect to the total mass of the first preparation, and the content of the non-conductive filler in the second preparation is, if contained in the second preparation, preferably 10% by mass or more with respect to the total mass of the second preparation.

Also preferably, the first preparation contains a non-conductive filler at a content of 10% by mass or more with respect to the total mass of the first preparation, and the second preparation contains a non-conductive filler at a content of 10% by mass or more with respect to the total mass of the second preparation.

### <Reducing Agent>

In the kit of the present disclosure for preparing a curable composition, at least one of the first preparation or the second preparation may contain a reducing agent.

Examples of the reducing agent include a sulfinic acid compound or a salt thereof, a hydrazine compound or a salt thereof, and an ascorbic acid compound or a salt thereof.

### (Sulfinic Acid Compound)

Examples of the sulfinic acid compound or salt thereof include an alkanesulfinic acid or a salt thereof, an alicyclic sulfinic acid or a salt thereof, and an aromatic sulfinic acid or a salt thereof.

Examples of the salt of a sulfinic acid compound include a lithium salt, a sodium salt, a potassium salt, a rubidium salt, a cesium salt, a magnesium salt, a calcium salt, a strontium salt, an iron salt, a zinc salt, an ammonium salt, a tetramethylammonium salt, and a tetraethylammonium salt.

Examples of the alkanesulfinic acid include methanesulfinic acid.

Examples of the alicyclic sulfinic acid include cyclohexanesulfinic acid and cyclooctanesulfinic acid.

Examples of the aromatic sulfinic acid include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, and naphthalenesulfinic acid.

In the present disclosure, when the first preparation or the second preparation contains a sulfinic acid compound, the first preparation or second preparation preferably contains the above-described heterocyclic amine compound, from the viewpoint of improving the storage stability.

The heterocyclic amine compound can reduce, during storage, unnecessary reactions caused by the sulfinic acid compound in the first preparation or second preparation.

Therefore, the configuration in which the first preparation or the second preparation contains a heterocyclic amine compound enables the first preparation or second preparation that contains a sulfinic acid compound to maintain storage stability.

When the first preparation or the second preparation contains a sulfinic acid compound, the heterocyclic amine compound preferably contains at least one of a secondary or tertiary amino group.

When the first preparation or the second preparation contains a sulfinic acid compound, the heterocyclic amine compound to be used may be an azole compound such as imidazole, triazole, mercaptotriazole, oxazole, oxadiazole, thiadiazole, tetrazole, benzimidazole, benzotriazole, hydroxybenzotriazole, mercaptobenzotriazole, benzoxazole, and benzothiadiazole.

Among them, benzimidazole and benzotriazole are preferable, and benzotriazole is more preferable, for use as the heterocyclic amine compound to be used in a case in which the first preparation or the second preparation contains a sulfinic acid compound.

### (Hydrazine Compound)

Examples of the hydrazine compound or a salt thereof include adipic acid dihydrazide, isophthalic acid dihydrazide, azelaic acid dihydrazide, acetohydrazide, benzoylhydrazine, tert-butyl 2-methylcarbazate, carbohydrazide, N,N'-dibenzoylhydrazine, malonic acid hydrazide, phthalic acid hydrazide, acetylphenylhydrazine, and salts of these hydrazine compounds.

### (Ascorbic Acid Compound)

Examples of the ascorbic acid compound or salt thereof include palmitoyl ascorbate.

### <Borate Compound>

The curable composition of the present disclosure may include a borate compound.

Inclusion of a borate compound in the curable composition of the present disclosure can further improve the polymerizability of the curable composition.

It should be noted that the above-described nitrogen-containing compound is excluded from the scope of the borate compound in the present disclosure.

For example, a compound that corresponds to the above-described nitrogen-containing compound and that is also a borate salt is classified into the above-described nitrogen-containing compound rather than the borate compound.

Examples of the borate compound include tetraalkylborate, tetraalkylammonium borate, and salts thereof.

Among the above, tetraalkylborates and salts thereof are preferable, tetraphenylborate and salts thereof are more preferable, and sodium tetraphenylborate is still more preferable for use as the borate compound.

In the present disclosure, at least one of the first preparation or the second preparation preferably contains at least one selected from the group consisting of a sulfinic acid compound and a borate compound.

In the kit of the present disclosure for preparing a curable composition, a configuration in which the first preparation contains a sulfonyl group-containing compound and in which the second preparation contains a nitrogen-containing compound is preferable from the viewpoint of improving storage stability.

In the kit of the present disclosure for preparing a curable composition, preferably, the second preparation contains sulfinic acid and nitrogen-containing compounds including an aromatic amine compound and a heterocyclic amine compound.

In the kit of the present disclosure for preparing a curable composition, a configuration in which the second preparation contains an aromatic amine compound, a heterocyclic amine compound, and sulfinic acid imparts higher storage stability than that in a case in which the second preparation does not contain a heterocyclic amine compound.

In the kit of the present disclosure for preparing a curable composition, the total content of organic peroxides contained in the first preparation and the second preparation is preferably 0.10% by mass or less (including a case in which the content is 0% by mass) with respect to the total mass of the curable composition to be prepared. A configuration in which the total content is 0.10% by mass or less can prevent deterioration of handleability caused by, for example, a phenomenon that the curable composition becomes heated during storage.

From the same viewpoint as that described above, the total content of organic peroxides contained in the first preparation and the second preparation is more preferably 0.05% by mass or less with respect to the total mass of the curable composition to be prepared.

The total content of organic peroxides contained in the first preparation and the second preparation may be more than 0% by mass, or 0.01% by mass or more, with respect to the total mass of the curable composition to be prepared.

### <Additive>

The curable composition of the present disclosure may contain additives such as a photopolymerization initiator, a stabilizer (polymerization inhibitor), a colorant, a fluorescent agent, and an ultraviolet absorber.

As the photopolymerization initiator, a known photopolymerization initiator can be used, and examples thereof include camphorquinone (CQ) and ethyl dimethylaminobenzoate (EDB).

An antimicrobial substance such as cetylpyridinium chloride, benzalkonium chloride, (meth)acryloyloxydecylpyridinium bromide, (meth)acryloyloxyhexadecylpyridinium chloride, (meth)acryloyloxydecylammonium chloride, or triclosan may be added.

Known dyes and pigments may be added to the curable composition of the present disclosure.

The kit of the present disclosure for preparing a curable composition is preferably a kit for a dental material.

### <Curable Composition>

The curable composition of the present disclosure includes the polymerization initiator of the present disclosure and a monomer. The curable composition of the present disclosure may have favorably improved polymerizability, by means of having the foregoing configuration.

Specific examples, preferred embodiments, and the like of the monomer in the curable composition are the same as those of the above-described monomers.

The curable composition of the present disclosure preferably further contains a non-conductive filler.

Specific examples, preferred embodiments, and the like of the non-conductive filler in the curable composition are the same as those of the non-conductive filler described above.

### <Cured Product>

The cured product of the present disclosure is a cured product of the curable composition of the present disclosure or a cured product obtained using the kit of the present disclosure for preparing a curable composition.

The cured product of the present disclosure can be suitably used as a dental material. That is, the dental material of the present disclosure preferably contains the cured product of the present disclosure.

### EXAMPLES

Examples according to the present disclosure are presented below. However, the present disclosure is not limited by the following examples.

The ingredients used in the examples are presented below.
<Monomer>
   dihydroxyethyl methacrylate trimethylhexyldicarbamate (molecular weight: 470) neopentyl glycol dimethacrylate (molecular weight: 240)
<Filler>
   barium aluminum silicate (non-conductive filler)
   silica dimethyl silylate (non-conductive filler, manufactured by Nippon Aerosil Co., Ltd., trade name "AEROSIL R 812")
<Transition Metal Compound>
   copper acetate
   copper ethylhexanoate
   acetylacetone copper
   copper (ii) bromide
   copper (ii) chloride
<Sulfinic Acid Compound>
   sodium p-toluenesulfinate
<Borate Compound>
   sodium tetraphenylborate
<Ascorbic Acid Compound>
   palmitoyl ascorbate
<Additive>
   diphenyliodonium hexafluorophosphate
<Nitrogen-containing Compound>
   dimethylaniline
   dimethyl-p-toluidine
   diethyl-p-toluidine
   diethanol-p-toluidine
   tetramethyl-1,3-phenylenediamine
   dimethylamino-1,4 phenylethanol
   tetraphenylborate dimethyl-p-toluidine
   tetraphenylborate tributylammonium salt
   tetraphenylborate triethanolammonium salt
   benzothiazole
   benzoxazole
   benzimidazole
   benzotriazole
<Sulfonyl group-containing Compound>
   saccharin
   sulfobenzoic acid anhydride
   benzodithiolone dioxide
   methyltosylbenzenesulfoamide
   saccharin sodium
   The structures of the sulfonyl group-containing compounds are shown below. 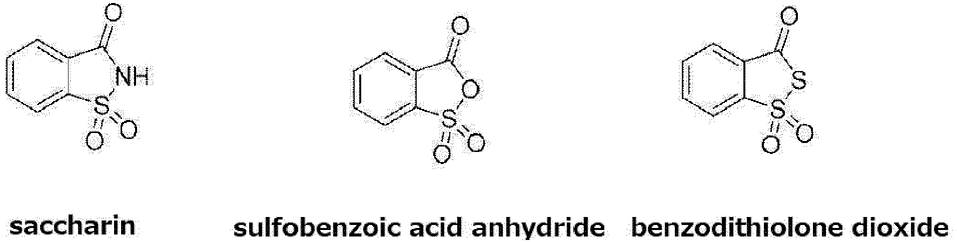 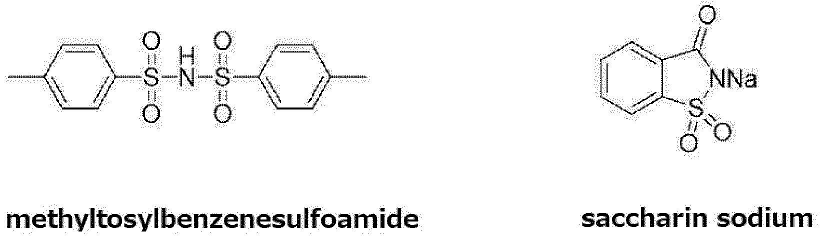
<Polymerization Inhibitor>
   MEHQ: methoxyphenol
   Di-tert-butylhydroxytoluene
<Peroxide>
   Benzoyl Peroxide

### [Examples 1 to 34 and Comparative Examples 1 to 4]

### <Evaluation of Monomer Polymerizability>

The first preparation and the second preparation of each of Examples 1 to 34 and Comparative Examples 1 to 4 were prepared using the components indicated in Tables 1 to 9.

The prepared first preparation and second preparation were mixed to prepare a curable composition of each of Examples 1 to 34 and Comparative Examples 1 to 4.

For each of the prepared curable compositions, monomer polymerizability was evaluated as follows.

Using DSC manufactured by RETSCH, the curable composition immediately after mixing was filled into an aluminum pan, the temperature inside the aluminum pan was rapidly adjusted to 37°C condition, and the curable composition was cured to produce a cured product. During this process, the temperature of the curable composition was measured over time, and the maximum temperature (highest peak value) of the curable composition and the time that elapsed until the temperature reached the maximum temperature (also referred to as the "maximum temperature reaching time") were measured. The results are indicated in Tables 1 to 9.

The temperature (°C) indicated at the heading "polymerizability" in Tables 1 to 9 means the maximum value of the temperature peaks of the curable composition. The lower the maximum value of the temperature peaks is, the better the polymerizability of the monomer in the curable composition is.

The time (minute) indicated at the heading "polymerizability" in Tables 1 to 9 means the time that had elapsed until the maximum value of the temperature peaks was obtained. The shorter the time that had elapsed until the maximum value of the temperature peaks was obtained is, the better the polymerizability of the monomer in the curable composition is.

### <Evaluation of Discoloring>

The color of the cured product produced in <Evaluation of Monomer Polymerizability> was visually checked. The results are shown in Tables 1 to 9.

### <Evaluation of Storage Stability>

The first preparation and the second preparation of each of Examples 31 to 34 and Comparative Example 4 prepared in <Evaluation of Monomer Polymerizability> were individually placed in a thermostatic chamber at 80°C and stored for 1 hour.

After the lapse of 1 hour, the time that had lapsed until the curable composition reached the maximum temperature (that is, the maximum temperature reaching time) was measured by the same method as the method described in <Evaluation of Monomer Polymerizability>.

The difference between the maximum temperature reaching time measured after storage and the maximum temperature reaching time measured without performing storage was calculated, and the storage stability was evaluated according to the following criteria. The results are shown in Table 9.

### Evaluation Criteria

A: The difference between the maximum temperature reaching time measured after storage and the maximum temperature reaching time measured without performing storage was less than 1 minute.
B: The difference between the maximum temperature reaching time measured after storage and the maximum temperature reaching time measured without performing storage was 1 minute or more.
C: Since a cured product was already found after storage, the maximum temperature reaching time could not be measured after storage.

**Table 1**

| | | Example 1 | | Example 2 | | Example 3 | | Example 4 | | Example 5 | |
|---|---|---|---|---|---|---|---|---|---|---|---|
| | | First preparation | Second preparation | First preparation | Second preparation | First preparation | Second preparation | First preparation | Second preparation | First preparation | Second preparation |
| Basic composition 1 | | 98.9900 | 99.0000 | 98.9900 | 99.0000 | 97.9900 | 99.0000 | 97.9900 | 99.0000 | 98.9987 | 98.0000 |
| Transition metal compound | Acetylacetone copper | 0.0100 | | 0.0100 | | 0.0100 | | 0.0100 | | 0.0013 | |
| Sulfinic acid compound | Sodium p-toluenesulfinate | | | | | | | 1.0000 | | | 1.0000 |
| Ascorbic acid compound | Palmitoyl ascorbate | | | | | | | | | | |
| Borate compound | Sodium tetraphenylborate | | | | | | | | | | |
| Nitrogen-containing compound | Dimethyl-p-toluidine | | 1.0000 | | | | 1.0000 | | 1.0000 | | |
| | Diethanol-p-toluidine | | | | | | | | | | 1.0000 |
| | Dimethylamino-1,4-phenylethanol | | | | | | | | | | |
| | Tetrametyl-1,3-phenylenediamine | | | | 1.0000 | | | | | | |
| | Benzothiazole | | | | | 1.0000 | | | | | |
| Sulfonyl group-containing compound | Saccharin | 1.0000 | | 1.0000 | | 1.0000 | | 1.0000 | | 1.0000 | |
| Additive | Diphenyliodonium hexafluorophosphate | | | | | | | | | | |
| Sum | | 100.0000 | 100.0000 | 100.0000 | 100.0000 | 100.0000 | 100.0000 | 100.0000 | 100.0000 | 100.0000 | 100.0000 |
| Polymerizability | Maximum temperature reaching time (min) | 2.4 | | 1.5 | | 2.7 | | 1.4 | | 1.7 | |
| | Maximum temperature (°C) | 38.6 | | 38.4 | | 37.9 | | 43.9 | | 41.7 | |
| Discoloring | | white | | white | | white | | white | | white | |

**Table 2**

| | | Example 6 | | Example 7 | | Example 8 | | Example 9 | | Example 10 | |
|---|---|---|---|---|---|---|---|---|---|---|---|
| | | First preparation | Second preparation | First preparation | Second preparation | First preparation | Second preparation | First preparation | Second preparation | First preparation | Second preparation |
| Basic composition 1 | | 98.9987 | 98.0000 | 97.9900 | 99.0000 | 97.9900 | 99.0000 | 98.9900 | 99.0000 | 97.9994 | 99.0000 |
| Transition metal compound | Acetylacetone copper | 0.0013 | | 0.0100 | | 0.0100 | | 0.0100 | | 0.0006 | |
| Sulfinic acid compound | Sodium p-toluenesulfinate | | 1.0000 | | | | | | | | |
| Ascorbic acid compound | Palmitoyl ascorbate | | | 1.0000 | | | | | | | |
| Borate compound | Sodium tetraphenylborate | | | | | 1.0000 | | | | 1.0000 | |
| Nitrogen-containing compound | Dimethyl-p-toluidine | | | | 1.0000 | | 1.0000 | | 1.0000 | | 1.0000 |
| | Diethanol-p-toluidine | | | | | | | | | | |
| | Dimethylamino-1,4-phenylethanol | | 1.0000 | | | | | | | | |
| | Tetramethyl-1,3-phenylenediamine | | | | | | | | | | |
| | Benzothiazole | | | | | | | | | | |
| Sulfonyl group-containing compound | Saccharin | 1.0000 | | 1.0000 | | 1.0000 | | 1.0000 | | 1.0000 | |
| Additive | Diphenyliodonium hexafluorophosphate | | | | | | | 1.0000 | | | |
| Sum | | 100.0000 | 100.0000 | 100.0000 | 100.0000 | 100.0000 | 100.0000 | 100.0000 | 100.0000 | 100.0000 | 100.0000 |
| Polymerizability | Maximum temperature reaching time (min) | 2.4 | | 3.5 | | 0.8 | | 2.2 | | 3.6 | |
| | Maximum temperature (°C) | 39.9 | | 37.9 | | 41.7 | | 37.9 | | 39.5 | |
| Discoloring | | white | | white | | white | | white | | white | |

**Table 3**

| | | Example 11 | | Example 12 | | Example 13 | | Example 14 | |
|---|---|---|---|---|---|---|---|---|---|
| | | First preparation | Second preparation | First preparation | Second preparation | First preparation | Second preparation | First preparation | Second preparation |
| Basic composition 1 | | 97.9950 | 99.0000 | 97.9950 | 99.0000 | 97.9950 | 99.0000 | 97.9950 | 99.0000 |
| Transition metal compound | Acetylacetone copper | 0.0050 | | 0.0050 | | 0.0050 | | 0.0050 | |
| | Copper acetate | | | | | | | | |
| | Copper ethylhexanoate | | | | | | | | |
| | Copper(II) bromide | | | | | | | | |
| | Copper(II) chloride | | | | | | | | |
| Sulfinic acid compound | Sodium p-toluenesulfinate | | | | | | | | |
| Borate compound | Sodium tetraphenylborate | 1.0000 | | 1.0000 | | 1.0000 | | 1.0000 | |
| Nitrogen-containing compound | Dimethyl-p-toluidine | | 1.0000 | | 1.0000 | | 1.0000 | | 1.0000 |
| | Tetraphenylborate dimethyl-p-toluidine | | | | | | | | |
| Sulfonyl group-containing compound | Saccharin | 1.0000 | | | | | | | |
| | Sulfobenzoic acid anhydride | | | 1.0000 | | | | | |
| | Benzodithiolone dioxide | | | | | 1.0000 | | | |
| | Methyltosylbenzenesulfoamide | | | | | | | 1.0000 | |
| Sum | | 100.0000 | 100.0000 | 100.0000 | 100.0000 | 100.0000 | 100.0000 | 100.0000 | 100.0000 |
| Polymerizability | Maximum temperature reaching time (min) | 1.5 | | 4.4 | | 5.0 | | 2.6 | |
| | Maximum temperature (°C) | 42.3 | | 37.5 | | 37.3 | | 42.7 | |
| Discoloring | | white | | white | | white | | white | |

**Table 4**

| | | Example 15 | | Example 16 | | Example 17 | | Example 18 | |
|---|---|---|---|---|---|---|---|---|---|
| | | First preparation | Second preparation | First preparation | Second preparation | First preparation | Second preparation | First preparation | Second preparation |
| Basic composition 1 | | 98.9987 | 98.0000 | 98.9987 | 98.0000 | 98.9987 | 98.0000 | 98.9987 | 98.0000 |
| Transition metal compound | Acetylacetone copper | | | | | | | | |
| | Copper acetate | 0.0013 | | | | | | | |
| | Copper ethylhexanoate | | | 0.0013 | | | | | |
| | Copper(II) bromide | | | | | 0.0013 | | | |
| | Copper(II) chloride | | | | | | | 0.0013 | |
| Sulfinic acid compound | Sodium p-toluenesulfinate | | | | | | | | |
| Borate compound | Sodium tetraphenylborate | | 1.0000 | | 1.0000 | | 1.0000 | | 1.0000 |
| Nitrogen-containing compound | Dimethyl-p-toluidine | | 1.0000 | | 1.0000 | | 1.0000 | | 1.0000 |
| | Tetraphenylborate dimethyl-p-toluidine | | | | | | | | |
| Sulfonyl group-containing compound | Saccharin | 1.0000 | | 1.0000 | | 1.0000 | | 1.0000 | |
| | Sulfobenzoic acid anhydride | | | | | | | | |
| | Benzodithiolone dioxide | | | | | | | | |
| | Methyltosylbenzenesulfoamide | | | | | | | | |
| Sum | | 100.0000 | 100.0000 | 100.0000 | 100.0000 | 100.0000 | 100.0000 | 100.0000 | 100.0000 |
| Polymerizability | Maximum temperature reaching time (min) | 2.6 | | 1.4 | | 1.2 | | 1.1 | |
| | Maximum temperature (°C) | 41.8 | | 43.5 | | 42.3 | | 43.2 | |
| Discoloring | | white | | white | | white | | white | |

**Table 5**

| | | Example 19 | | Example 20 | | Example 21 | |
|---|---|---|---|---|---|---|---|
| | | First preparation | Second preparation | First preparation | Second preparation | First preparation | Second preparation |
| Basic composition 1 | | 98.9987 | 98.0000 | 98.9987 | 98.0000 | 98.9987 | 98.0000 |
| Transition metal compound | Acetylacetone copper | 0.0013 | | 0.0013 | | 0.0013 | |
| | Copper acetate | | | | | | |
| | Copper ethylhexanoate | | | | | | |
| | Copper(II) bromide | | | | | | |
| | Copper(II) chloride | | | | | | |
| Sulfinic acid compound | Sodium p-toluenesulfinate | | | | 1.0000 | | |
| Borate compound | Sodium tetraphenylborate | | 1.0000 | | | | |
| Nitrogen-containing compound | Dimethyl-p-toluidine | 1.0000 | | 1.0000 | | | |
| | Tetraphenylborate dimethyl-p-toluidine | | | | | | 2.0000 |
| Sulfonyl group-containing compound | Saccharin | | 1.0000 | | 1.0000 | 1.0000 | |
| | Sulfobenzoic acid anhydride | | | | | | |
| | Benzodithiolone dioxide | | | | | | |
| | Metthyltosylbenzenesulfoamide | | | | | | |
| Sum | | 100.0000 | 100.0000 | 100.0000 | 100.0000 | 100.0000 | 100.0000 |
| Polymerizability | Maximum temperature reaching time (min) | 2.1 | | 2.0 | | 4.8 | |
| | Maximum temperature (°C) | 42.6 | | 43.5 | | 40.2 | |
| Discoloring | | white | | white | | white | |

**Table 6**

| | | Example 22 | | Example 23 | | Example 24 | | Example 25 | | Example 26 | |
|---|---|---|---|---|---|---|---|---|---|---|---|
| | | First preparation | Second preparation | First preparation | Second preparation | First preparation | Second preparation | First preparation | Second preparation | First preparation | Second preparation |
| Basic composition 1 | | 98.8987 | 98.0000 | 98.8987 | 98.0000 | 98.9987 | 97.9000 | 98.8987 | 98.0000 | 98.8987 | 98.0000 |
| Transition metal compound | Acetylacetone copper | 0.0013 | | 0.0013 | | 0.0013 | | 0.0013 | | 0.0013 | |
| Sulfinic acid compound | Sodium p-toluenesulfinate | | | | 1.0000 | | | | | | |
| Borate compound | Sodium tetraphenylborate | | 1.0000 | | | | 1.0000 | | | | |
| Nitrogen-containing compound | Dimethylaniline | | | | | | | | | | |
| | Dimethyl-p-toluidine | 1.0000 | | | 1.0000 | | | | 1.0000 | | 1.0000 |
| | Diethyl-p-toluidine | | | | | 1.0000 | | | | | |
| | Tetraphenylborate tributyl ammonium salt | | | | | | | | 1.0000 | | |
| | Tetraphenylborate triethanol ammonium salt | | | | | | | | | | 1.0000 |
| Sulfonyl group-containing compound | Saccharin | | | | | | | 1.0000 | | 1.0000 | |
| | Saccharin sodium | | 1.0000 | 1.0000 | | | 1.0000 | | | | |
| Polymerization inhibitor | Methoxyphenol | 0.1000 | | 0.1000 | | | 0.1000 | 0.1000 | | 0.1000 | |
| Sum | | 100.0000 | 100.0000 | 100.0000 | 100.0000 | 100.0000 | 100.0000 | 100.0000 | 100.0000 | 100.0000 | 100.0000 |
| Polymerizability | Maximum temperature reaching time (min) | 2.8 | | 1.9 | | 4.5 | | 1.5 | | 2.0 | |
| | Maximum temperature (°C) | 41.7 | | 39.6 | | 38.8 | | 45.4 | | 43.5 | |
| Discoloring | | white | | white | | white | | white | | white | |

**Table 7**

| | | Example 27 | | Example 28 | | Example 29 | | Example 30 | |
|---|---|---|---|---|---|---|---|---|---|
| | | First preparation | Second preparation | First preparation | Second preparation | First preparation | Second preparation | First preparation | Second preparation |
| Basic composition 1 | | 98.9980 | 97.0000 | 98.9980 | 97.0000 | 98.9980 | 97.0000 | 98.9988 | 97.0000 |
| Transition metal compound | Acetylacetone copper | 0.0020 | | 0.0020 | | 0.0020 | | 0.0013 | |
| Sulfinic acid compound | Sodium p-toluenesulfinate | | 1.0000 | | 1.0000 | | 1.0000 | | 1.0000 |
| Nitrogen-containing compound | Diethanol-p-toluidine | | 1.0000 | | 1.0000 | | 1.0000 | | 1.0000 |
| | Benzothiazole | | 1.0000 | | | | | | |
| | Benzoxazole | | | | 1.0000 | | | | |
| | Benzimidazole | | | | | | 1.0000 | | |
| | Benzotriazole | | | | | | | | 1.0000 |
| Sulfonyl group-containing compound | Saccharin | 1.0000 | | 1.0000 | | 1.0000 | | 1.0000 | |
| Polymerization inhibitor | Methoxyphenol | | | | | | | 0.1000 | |
| Sum | | 100.0000 | 100.0000 | 100.0000 | 100.0000 | 100.0000 | 100.0000 | 100.0000 | 100.0000 |
| Polymerizability | Maximum temperature reaching time (min) | 1.2 | | 1.7 | | 3.5 | | 1.3 | |
| | Maximum temperature (°C) | 42.9 | | 40.4 | | 39.7 | | 40.5 | |
| Discoloring | | white | | white | | white | | white | |

**Table 8**

| | | Comparative Example 1 | | Comparative Example 2 | | Comparative Example 3 | | Comparative Example 4 | |
|---|---|---|---|---|---|---|---|---|---|
| | | First preparation | Second preparation | First preparation | Second preparation | First preparation | Second preparation | First preparation | Second preparation |
| Basic composition 1 | | 99.9900 | 99.0000 | 99.0000 | 99.0000 | 99.9900 | 99.0000 | 99.0000 | 99.0000 |
| Transition metal compound | Acetylacetone copper | 0.0100 | | | | 0.0100 | | | |
| Sulfinic acid compound | Sodium p-toluenesulfinate | | | | | | | | |
| Borate compound | Sodium tetraphenylborate | | | | | | | | |
| Nitrogen-containing compound | Dimethylaniline | | | | | | | | 1.0000 |
| | Dimethyl-p-toluidine | | | | 1.0000 | | 1.0000 | | |
| | Diethyl-p-toluidine | | | | | | | | |
| | Tetraphenylborate tributyl ammonium salt | | | | | | | | |
| | Tetraphenylborate triethanol ammonium salt | | | | | | | | |
| Sulfonyl group-containing compound | Saccharin | | 1.0000 | 1.0000 | | | | | |
| | Saccharin sodium | | | | | | | | |
| Polymerization inhibitor | Methoxyphenol | | | | | | | | |
| Peroxide | Benzoyl peroxide | | | | | | | 1.0000 | |
| Sum | | 100.0000 | 100.0000 | 100.0000 | 100.0000 | 100.0000 | 100.0000 | 100.0000 | 100.0000 |
| Polymerizability | Maximum temperature reaching time (min) | no peak | | no peak | | no peak | | 1.1 | |
| | Maximum temperature (°C) | - | | - | | - | | 40.9 | |
| Discoloring | | - | | - | | - | | yellow | |

**Table 9**

| | | Example 31 | | Example 32 | | Example 33 | | Example 34 | | Comparative Example 4 | |
|---|---|---|---|---|---|---|---|---|---|---|---|
| | | First preparation | Second preparation | First preparation | Second preparation | First preparation | Second preparation | First preparation | Second preparation | First preparation | Second preparation |
| Basic composition 1 | | 98.9980 | 97.0000 | 98.9980 | 97.0000 | 98.9980 | 97.0000 | 98.8988 | 97.0000 | 99.0000 | 99.0000 |
| Transition metal compound | Acetylacetone copper | 0.0020 | | 0.0020 | | 0.0020 | | 0.0013 | | | |
| Sulfinic acid compound | Sodium p-toluenesulfinate | | 1.0000 | | 1.0000 | | 1.0000 | | 1.0000 | | |
| Nitrogen-containing compound | Dimethylamino-1,4-phenylethanol | | 1.0000 | | 1.0000 | | 1.0000 | | 1.0000 | | |
| | Dimethylaniline | | | | | | | | | | 1.0000 |
| | Benzothiazole | | 1.0000 | | | | | | | | |
| | Benzoxazole | | | | 1.0000 | | | | | | |
| | Benzimidazole | | | | | | 1.0000 | | | | |
| | Benzotriazole | | | | | | | | 1.0000 | | |
| Sulfonyl group-containing compound | Saccharin | 1.0000 | | 1.0000 | | 1.0000 | | 1.0000 | | | |
| Polymerization inhibitor | Methoxyphenol | | | | | | | 0.1000 | | | |
| Peroxide | Benzoyl peroxide | | | | | | | | | 1.0000 | |
| Sum | | 100.0000 | 100.0000 | 100.0000 | 100.0000 | 100.0000 | 100.0000 | 100.0000 | 100.0000 | 100.0000 | 100.0000 |
| Polymerizability | Maximum temperature reaching time (min) | 1.2 | | 1.3 | | 3.7 | | 2.2 | | 1.1 | |
| | Maximum temperature (°C) | 40.1 | | 43.6 | | 41.4 | | 41.0 | | 40.9 | |
| Discoloring | | white | | white | | white | | white | | yellow | |
| Storage stability | Maximum temperature reaching time (min) measured after storage | 1.2 | | 1.3 | | 5.2 | | 1.9 | | unmeasureable | |
| | Maximum temperature (°C) measured after storage | 41.8 | | 43.9 | | 38.9 | | 43.0 | | | |
| | Evaluation | A | | A | | B | | A | | C | |

The numbers indicated in the columns for the respective compositions or ingredients in Tables 1 to 9 refer to the amounts (parts by mass) of the compositions or ingredients with respect to the total mass of the first or second preparation.

"Basic composition 1" in Tables 1 to 9 refers to a composition common to the Examples and Comparative Examples that contain basic composition 1. The ingredients and contents thereof in basic composition 1 are as indicated in Table 10.

**Table 10**

| Basic composition 1 | | % by mass |
|---|---|---|
| Monomer | Dihydroxyethyl methacrylate trimethylhexyl dicarbamate | 29.58 |
| | Neopentyl glycol dimethacrylate | 7.14 |
| Filler | Barium aluminum silicate | 61.21 |
| | Silica dimethyl silylate | 2.04 |
| Polymerization inhibitor | Di-tert-butylhydroxytoluene | 0.03 |
| Sum | | 100 |

The numbers indicated in the columns for the basic composition or the ingredients in Table 10 refer to the amounts (parts by mass) of the composition or the ingredients with respect to the total mass of the basic composition.

As is seen in Tables 1 to 9, it was demonstrated that the Examples, in which a polymerization initiator that contained a transition metal compound, a nitrogen-containing compound, and a sulfonyl group-containing compound was used, had excellent monomer polymerizability, as indicated by the short length of time and the high temperature associated with the curing. In addition, it was also demonstrated that the Examples had suitability for dental materials, as indicated by reduced discoloration.

In contrast, in Comparative Example 1, in which a polymerization initiator that did not contain a nitrogen-containing compound was used, Comparative Example 2, in which a polymerization initiator that did not contain a transition metal compound was used, and Comparative Example 3, in which a polymerization initiator that did not contain a sulfonyl group-containing compound was used, polymerization did not proceed, and a cured product was not obtained.

In Comparative Example 4, in which a polymerization initiator that contained a peroxide but did not contain a transition metal compound and a sulfonyl group-containing compound was used, a cured product of which color had changed to yellow was obtained.

As seen in Table 9, Examples 31 to 34, in which a sulfinic acid compound and a heterocyclic aromatic compound as a nitrogen-containing compound were contained, exhibited a superior storage stability, compared to Comparative Example 4, in which a sulfinic acid compound and a heterocyclic aromatic compound were not contained.

In particular, Examples 31,33, and 34, which contained benzothiazole, benzoxazole, or benzotriazole as a heterocyclic aromatic compound, exhibited more improved storage stability.

### [Examples 35 to 37]

### <Evaluation of Monomer Polymerizability>

A first preparation and a second preparation for each of Examples 35 to 37 were prepared using the ingredients indicated in Table 11.

The prepared first preparation and second preparation were mixed to prepare a curable composition of each of Examples 35 to 37.

Each of the prepared curable compositions was subjected to evaluation of monomer polymerizability according to the same method as the method described in the item starting with the heading "<Evaluation of Monomer Polymerizability>" described above. In addition, the discoloring of a cured product produced was evaluated according to the same method as the method described in the item starting with the heading "<Evaluation of Discoloring>" described above. The results are indicated in Table 11.

### <Evaluation of Adhesiveness>

A cow mandiblar anterior tooth was subjected to tooth root cutting and pulpectomy. This tooth was placed in a plastic cylindrical container having a diameter of 25 mm and a depth of 25 mm, and embedded in an acrylic resin. The resultant product will be also referred to as a cow tooth for adhesion. The cow tooth for adhesion was polished with water-resistant emery paper (P 400) immediately before use, and the smooth surface of the cow tooth dentin was exposed by polishing and used. The first preparation and the second preparation of each of the Examples and Comparative Examples prepared in the <Evaluation of Monomer Polymerizability> were used as test samples to be used in the measurement of adhesive power, and adhesiveness was evaluated in accordance with ISO 16506.

Specifically, excessive moisture on the adherend surface of the cow tooth for adhesion was wiped off with a Kimwipe, and one drop of water was dripped on the adherend surface. Next, the first preparation and the second preparation indicated in Table 11 were mixed with each other for 20 seconds, and then, an appropriate amount thereof was applied to an adherend surface of a cured product having a cylindrical column shape that had been prepared, in advance, from a filler composition (VINUS DIAMOND, manufactured by Kulzer; the adherend surface thereof having been polished with water-resistant emery paper P 180 in advance). Then, the cured product was mounted vertically on the cow tooth for adhesion. Thereafter, adhesion was performed by applying 5 N force, using a dedicated jig.

After the surplus portion of the applied composition was removed, adhesive power was measured: after the adhered items were left to stand still at 37°C for 90 minutes, in the case of chemical polymerization; or after the adhered items were irradiated with light from 2 directions, for 10 seconds for each direction, using an LED visible light irradiator (TRANSLUX 2WAVE, manufactured by Kulzer) so as to cause curing, and then left to stand still at 37°C for 30 minutes, in the case of photopolymerization,

A shear load was applied, at a crosshead speed of 1.0 mm/min, in a direction parallel to the cow tooth for adhesion and in contact with the surface of the cow tooth, and the adhesion power was determined from the shear load at the time when the cylindrical cured body adhered to the cow tooth for adhesion was separated from the surface of the cow tooth. The results are indicated in Table 11.

**Table 11**

| | | Example 33 | | Example 36 | | Example 37 | |
|---|---|---|---|---|---|---|---|
| | | First preparation | Second preparation | First preparation | Second preparation | First preparation | Second preparation |
| Basic composition 2 | | | 97.0000 | | 97.0000 | | |
| Basic composition 3 | | | | 79.0000 | | | |
| Basic composition 4 | | 79.0000 | | | | 86.0000 | |
| Basic composition 5 | | | | | | | 96.0000 |
| Acidic compound | MDP | 20.0000 | | 20.0000 | | 10.0000 | |
| Transition metal compound | Acetylacetone copper | 0.0008 | | 0.8000 | | 0.5000 | |
| Photopolymerization initiator | Camphor quinone | 0.1000 | | 0.1000 | | 0.1000 | |
| | Dimethylaminobutoxy benzoate | | 0.1000 | | 0.1000 | | 0.1000 |
| Sulfonyl group-containing compound | Saccharin | 1.0000 | | 1.0000 | | 1.0000 | |
| Sulfmic acid compound | Sodium p-toluenesulfinate | | 1.0000 | | 1.0000 | | 1.0000 |
| Inorganic peroxide | Sodium persulfate | 1.0000 | | 1.0000 | | 2.0000 | |
| Nitrogen-containing compound | Diethanol-p-toluidine | | 1.0000 | | 1.0000 | | 1.0000 |
| | Benzoxazole | | 1.0000 | | 1.0000 | | 1.0000 |
| Sum | | 101.1008 | 100.1000 | 101.9000 | 100.1000 | 99.6000 | 99.1000 |
| Polymerizability | Maximum temperature reaching time (min) | 2.8 | | 3.0 | | 1.5 | |
| | Maximum temperature (°C) | 42.8 | | 41.5 | | 41.7 | |
| Discoloring | | white | | white | | white | |
| Adhesiveness | Shear stress (chemical polymerization) (MPa) | 6.1 ± 4.4 | | 8.0 ± 6.3 | | 7.9 ± 3.7 | |
| | Shear stress (photopolymerization) (MPa) | N.D. | | 11.5 ± 6.1 | | N.D. | |

In Table 11, details of each item are as follows.

### MDP: 10-(meth)acryloyloxydecyl dihydrogen phosphate

Each of basic compositions 2 to 5 in Table 11 is a composition common to Examples and Comparative Examples that contain the designated basic composition. The ingredients and contents thereof in basic compositions 2 to 5 are as indicated in Tables 12 to 15.

**Table 12**

| Basic composition 2 | | % by mass |
|---|---|---|
| Polymerization inhibitor | Di-tert-butylhydroxytoluene | 0.028 |
| Monomer | Ethoxylated bisphenol A dimethacrylate | 23.833 |
| | Neopentyl glycol dimethacrylate | 8.256 |
| Filler | Aluminum oxide | 2.752 |
| | Barium aluminum silicate | 65.132 |
| Sum | | 100 |

**Table 13**

| Basic composition 3 | | % by mass |
|---|---|---|
| Polymerization inhibitor | Di-tert-butylhydroxytoluene | 0.050 |
| Monomer | Ethoxylated bisphenol A dimethacrylate | 10.995 |
| | Bisphenol A dimethacrylate | 17.491 |
| | Hydroxyethyl methacrylate | 6.497 |
| Filler | Nano silica | 4.998 |
| | Silica | 59.970 |
| Sum | | 100 |

**Table 14**

| Basic composition 4 | | % by mass |
|---|---|---|
| Polymerization inhibitor | Di-tert-butylhydroxytoluene | 0.049 |
| Monomer | Bisphenol A dimethacrylate | 17.318 |
| | Hydroxyethyl methacrylate | 6.432 |
| Filler | Nano silica | 4.948 |
| | Silica | 71.252 |
| Sum | | 100 |

**Table 15**

| Basic composition 5 | | % by mass |
|---|---|---|
| Polymerization inhibitor | Di-tert-butylhydroxytoluene | 0.030 |
| Monomer | Ethoxylated bisphenol A dimethacrylate | 23.970 |
| | Triethylene glycol dimethacrylate | 8.500 |
| Filler | Aluminum oxide | 2.500 |
| | Barium aluminum silicate | 65.000 |
| Sum | | 100 |

As demonstrated in Table 11, Examples regarding a curable composition that contains an acidic compound exhibited excellent adhesiveness.

### [Examples 38 to 43]

### <Evaluation of Monomer Polymerizability>

The first preparation and the second preparation of each of Examples 38 to 43 were prepared using the ingredients indicated in Tables 16 and 17.

The prepared first preparation and second preparation were mixed to prepare a curable composition of each of Examples 38 to 43.

Each of the curable compositions prepared was subjected to evaluation of monomer polymerizability according to the same method as the method described in the item starting with the heading "<Evaluation of Monomer Polymerizability>" described above.

### <Evaluation of Storage Stability 2>

The first preparation and the second preparation indicated in Tables 16 and 17, which were prepared in <Evaluation of Monomer Polymerizability>, were individually placed in a thermostatic bath at 80°C, and stored for the length of time indicated in the table.

After the lapse of the storage time, the time that had lapsed until the curable composition reached the maximum temperature (that is, the maximum temperature reaching time) was measured by the same method as the method described in <Evaluation of Monomer Polymerizability>.

The difference between the maximum temperature reaching time measured after storage and the maximum temperature reaching time measured without performing storage was calculated, and the storage stability was evaluated according to the following criteria.
A: The difference between the maximum temperature reaching time measured after storage and the maximum temperature reaching time measured without performing storage was less than 1 minute.
B: The difference between the maximum temperature reaching time measured after storage and the maximum temperature reaching time measured without performing storage was 1 minute or more.
C: Since a cured product was already formed after storage, the maximum temperature reaching time after storage could not be measured.

**Table 16**

| | | | Example 38 | | Example 39 | | Example 40 | |
|---|---|---|---|---|---|---|---|---|
| | | | First preparation | Second preparation | First preparation | Second preparation | First preparation | Second preparation |
| Basic composition 1 | | | 98.8000 | 97.0000 | 98.8000 | 97.0000 | 98.8000 | 97.0000 |
| Transition metal compound | Acetylacetone copper | | 0.0020 | | 0.0020 | | 0.0020 | |
| Sulfonyl group-containing compound | Saccharin | | 1.0000 | | 1.0000 | | 1.0000 | |
| Sulfinic acid compound | Sodium p-toluenesulfinate | | | 1.0000 | | 1.0000 | | 1.0000 |
| Nitrogen-containing compound | Diethanol-p-toluidine | | | 1.0000 | | | | |
| | Dimethylamino-1,4-phenylethanol | | | | | 1.0000 | | |
| | Dimethyl-p-toluidine | | | | | | | 1.0000 |
| | Benzoxazole | | | 1.0000 | | 1.0000 | | 1.0000 |
| Sum | | | 99.8020 | 100.0000 | 99.8020 | 100.0000 | 99.8020 | 100.0000 |
| Polymerizability | Measured before storage | Maximum temperature reaching time (min) | 1.7 | | 1.3 | | 1.0 | |
| | | Maximum temperature (°C) | 40.4 | | 43.6 | | 43.6 | |
| Storage stability 2 | 80°C, 0.5 hours | Max. temp reaching time (min) measured after storage | 1.2 | | 1.6 | | 1.1 | |
| | | Max. temp (°C) measured after storage | 43.2 | | 43.4 | | 44.0 | |
| | 80°C, 1.0 hour | Max. temp reaching time (min) measured after storage | 1.2 | | 1.3 | | 1.0 | |
| | | Max. temp. (°C) measured after storage | 42.7 | | 43.9 | | 41.5 | |
| | 80°C, 2.0 hours | Max. temp reaching time (min) measured after storage | 1.1 | | 1.2 | | 0.9 | |
| | | Max. temp (°C) measured after storage | 44.8 | | 44.8 | | 43.5 | |
| | 80°C, 3.0 hours | Max. temp reaching time (min) measured after storage | 1.2 | | 1.4 | | 1.1 | |
| | | Max. temp (°C) measured after storage | 44.6 | | 42.1 | | 42.1 | |
| | 80°C, 4.0 hours | Max. temp reaching time (min) measured after storage | 1.1 | | 1.4 | | 1.1 | |
| | | Max. temp (°C) measured after storage | 43.8 | | 42.9 | | 43.2 | |
| | 80°C, 5.0 hours | Max. temp reaching time (min) measured after storage | 1.1 | | unmeasureable | | unmeasureable | |
| | | Max. temp (°C) measured after storage | 45.2 | | | | | |

**Table 17**

| | | | Example 39 | | Example 41 | | Example 42 | | Example 43 | |
|---|---|---|---|---|---|---|---|---|---|---|
| | | | First preparation | Second preparation | First preparation | Second preparation | First preparation | Second preparation | First preparation | Second preparation |
| Basic composition 1 | | | 98.8000 | 97.0000 | 98.8000 | 97.0000 | 98.8000 | 97.0000 | 98.7750 | 97.0000 |
| Polymerization inhibitor | Methoxy-phenol | | | | | | | | 0.1000 | |
| Transition metal compound | Acetylacetone copper | | 0.0020 | | 0.0020 | | 0.0020 | | 0.0013 | |
| Sulfonyl group-containing compound | Saccharin | | 1.0000 | | 1.0000 | | 1.0000 | | 1.0000 | |
| Sulfinic acid compound | Sodium p-toluenesulfinate | | | 1.0000 | | 1.0000 | | 1.0000 | | 1.0000 |
| Nitrogen-containing compound | Dimethylamino-1,4-phenylethanol | | | 1.0000 | | 1.0000 | | 1.0000 | | 1.0000 |
| | Benzoxazole | | | 1.0000 | | | | | | |
| | Benzothiazole | | | | | 1.0000 | | | | |
| | Benzimidazole | | | | | | | 1.0000 | | |
| | Benzotriazole | | | | | | | | | 1.0000 |
| Sum | | | 99.8020 | 100.0000 | 99.8020 | 100.0000 | 99.8020 | 100.0000 | 99.8763 | 100.0000 |
| Polymerizability | Measured before! storage | Max. temp reaching time (min) | 1.3 | | 1.2 | | 3.7 | | 2.2 | |
| | | Max. temp. (°C) | 43.6 | | 40.1 | | 41.4 | | 41.0 | |
| | 80°C, 0.5 hours | Max. temp. reaching time (min) measured after storage | 1.6 | | 1.1 | | 5.2 | | 1.5 | |
| | | Max. temp. (°C) measured after storage | 43.4 | | 40.5 | | 39.1 | | 41.4 | |
| | 80°C, 1.0 hour | Max. temp. reaching time (min) measured after storage | 1.3 | | 1.2 | | 5.2 | | 1.9 | |
| | | Max. temp. (°C) measured after storage | 43.9 | | 41.8 | | 38.9 | | 43.0 | |
| | 80°C, 2.0 hours | Max. temp. reaching time (min) measured after storage | 1.2 | | 1.0 | | unmeasureable | | 3.2 | |
| Storage stability 2 | | Max. temp. (°C) measured after storage | 44.8 | | 43.0 | | | | 40.8 | |
| | 80°C. 3.0 hours | Max. temp, reaching time (min) measured after storage | 1.4 | | 1.1 | | unmeasureable | | 3.9 | |
| | | Max. temp. (°C) measured after storage | 42.1 | | 43.3 | | | | 37.9 | |
| | 80°C, 4.0 hours | Max. temp. reaching time (min) measured after storage | 1.4 | | 1.1 | | unmeasureable | | 4.1 | |
| | | Max. temp. (°C) measured after storage | 42.9 | | 42.2 | | | | 38.1 | |
| | 80°C 5.0 hours | Max. temp. reaching time (min) measured after storage | unmeasureable - | | 1.0 | | unmeasureable | | unmeasureable | |
| | | Max. temp. (°C) measured after storage | | | 43.5 | | | | | |

As demonstrated in Tables 16 and 17, Examples in which the curable composition of the present disclosure was used exhibited excellent storage stability under thermal load of 80°C.

In each of the Examples indicated in Tables 16 and 17, the color of the cured product was visually observed and found to be white. This means that reduction of discoloration of the cured product could be achieved.

The disclosure of Japanese Patent Application No. 2020-121716, filed July 15, 2020, is incorporated herein by reference in its entirety.

All documents, patent applications, and technical standards mentioned in this specification are incorporated herein by reference to the same extent as if each document, patent application, or technical standard were specifically and individually indicated to be incorporated by reference.

## Claims

1. A polymerization initiator, comprising a transition metal compound, a nitrogen-containing compound, and a sulfonyl group-containing compound.

2. The polymerization initiator according to claim 1, wherein the sulfonyl group-containing compound contains an aromatic ring.

3. The polymerization initiator according to claim 1 or 2, wherein the sulfonyl group-containing compound has a structure represented by the following Formula (1): 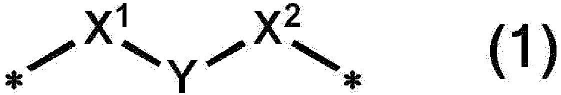
wherein, in Formula (1), each of X¹ and X² is independently a sulfonyl group or a carbonyl group, and at least one of X¹ or X² is a sulfonyl group, and
wherein, in Formula (1), Y is an oxygen atom, -NH-, -NNa-, or a sulfur atom, and * represents a bonding position.

4. The polymerization initiator according to any one of claims 1 to 3, wherein the transition metal compound comprises a copper compound.

5. The polymerization initiator according to any one of claims 1 to 4, wherein the nitrogen-containing compound comprises an aromatic amine compound.

6. The polymerization initiator according to claim 5, wherein the nitrogen-containing compound further comprises a heterocyclic amine compound.

7. The polymerization initiator according to claim 6, wherein the heterocyclic amine compound is a compound represented by the following Formula (2): 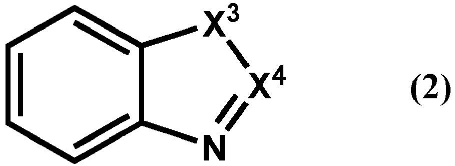 wherein, in Formula (2), X³ is an oxygen atom, a sulfur atom, or a nitrogen atom, and X⁴ is -N= or -CH=.

8. A kit for preparing a curable composition, comprising:
a first preparation containing a monomer (A); and
a second preparation containing a monomer (B),
wherein each of the transition metal compound, the nitrogen-containing compound, and the sulfonyl group-containing compound contained in the polymerization initiator according to any one of claims 1 to 7 is independently contained in at least one of the first preparation or the second preparation.

9. The kit for preparing a curable composition according to claim 8, wherein the first preparation contains the sulfonyl group-containing compound, and the second preparation contains the nitrogen-containing compound.

10. The kit for preparing a curable composition according to claim 8 or 9, wherein the second preparation contains the nitrogen-containing compound and sulfinic acid, and the nitrogen-containing compound includes an aromatic amine compound and a heterocyclic amine compound.

11. The kit for preparing a curable composition according to any one of claims 8 to 10, wherein a total content of organic peroxides contained in the first preparation and the second preparation is 0.10% by mass or less with respect to a total mass of the curable composition to be prepared.

12. The kit for preparing a curable composition according to any one of claims 8 to 11, wherein at least one of the first preparation or the second preparation contains a non-conductive filler.

13. The kit for preparing a curable composition according to claim 12, wherein the first preparation contains the non-conductive filler, and a content of the non-conductive filler is 10% by mass or more with respect to a total mass of the first preparation, and the second preparation includes the non-conductive filler, and a content of the non-conductive filler is 10% by mass or more with respect to a total mass of the second preparation.

14. The kit for preparing a curable composition according to any one of claims 8 to 13, wherein the monomer (A) and the monomer (B) include a (meth)acrylic monomer (C) having a molecular weight of 100 to 2000.

15. The kit for preparing a curable composition according to claim 14, wherein a content of the (meth)acrylic monomer (C) with respect to a total content of the monomer (A) and the monomer (B) is 50% by mass or more.

16. The kit for preparing a curable composition according to any one of claims 8 to 15, which is for a dental material.

17. A curable composition, comprising the polymerization initiator according to any one of claims 1 to 7, and a monomer.

18. A cured product of the curable composition according to claim 17.

19. A dental material, comprising the cured product according to claim 18.
